# EUROPEAN PATENT SPECIFICATION

(11) **EP 1 214 298 B1**
(45) Date of publication and mention of the grant of the patent: **30.05.2012**
(21) Application number: 00963531.9
(22) Date of filing: 15.09.2000
(51) Int. Cl.: C07D 209/12, A61K 31/404, A61P 43/00, C07F 9/572, C07F 9/24

(54) **INDOLE-CONTAINING AND COMBRETASTATIN-RELATED ANTI-MITOTIC AND ANTI-TUBULIN POLYMERIZATION AGENTS**
INDOL-ENTHALTENDE UND COMBRESTATIN-ANVERWANDTE ANTI-MITOTISCHE UND DIE TUBULIN-POLYMERISATION HEMMENDE WIRKSTOFFE
AGENTS DE POLYMERISATION ANTIMITOTIQUES ET ANTITUBULINES CONTENANT DE L'INDOLE ET APPARENTES A LA COMBRETASTATINE

(30) Priority: 17.09.1999 US 154639 P
(43) Date of publication of application: 19.06.2002
(73) Proprietor: BAYLOR UNIVERSITY, Waco, TX 78798-7034 (US)
(72) Inventor: PINNEY, Kevin, G., Hewitt, TX 76643 (US); WANG, Feng, King of Prussia, PA 19406 (US); DEL PILAR MEJIA, Maria, Des Plaines, IL 60016 (US)
(74) Representative: Crump, Julian Richard John
(86) International application number: PCT/US2000/025408
(87) International publication number: WO 2001/019794

(56) References cited:
- WO-A-00/48606
- WO-A-98/39323
- WO-A-99/34788

## Description

Tubulin is currently among the most attractive therapeutic targets in new drug design for the treatment of solid tumors.^{1c} The heralded success of vincristine and taxol along with the promise of combretastatin A-4 (CA-4) prodrug and dolastatin 10, to name just a few, have firmly established the clinical efficacy of these antimitotic agents for cancer treatment.

An aggressive chemotherapeutic strategy toward the treatment of solid-tumor cancers continues to rely on the development of architecturally new and biologically more potent anti-tumor, anti-mitotic agents which mediate their effect through a direct binding interaction with tubulin. A variety ofclinically-promising compounds which demonstrate potent cytotoxicity and antitumor activity are known to effect their primary mode of action through an efficient inhibition of tubulin polymerization. This class of compounds undergoes an initial interaction (binding) to the ubiquitous protein tubulin which in turn arrests the ability of tubulin to polymerize into microtubules which are essential components for cell maintenance and division.² During metaphase of the cell cycle, the nuclear membrane is broken down and the cytoskeletal protein tubulin is able to form centrosomes (also called microtubule organizing centers) and through polymerization and depolymerization of tubulin the dividing chromosomes are separated. Currently, the most recognized and clinically useful members of this class of antimitotic, antitumor agents are vinblastine and vincristine³ along with taxol.⁴ Additionally, the natural products rhizoxin,⁵ combretastatin A-4 and A-2,⁶ curacin A,¹ podophyllotoxin,⁷ epothilones A and B,⁸ dolastatin 10⁹ and welwistatin¹⁰ (to name just a few) as well as certain synthetic analogues including phenstatin,¹¹ the 2-styrylquinazolin-4(3H)-ones (SQO),¹² and highly oxygenated derivatives of *cis*- and *trans*-stilbene¹³ and dihydrostilbene are all known to mediate their cytotoxic activity through a binding interaction with tubulin. The exact nature of this binding site interaction remains largely unknown, and definitely varies between the series of compounds. Photoaffinity labeling and other binding site elucidation techniques have identified several key binding sites on tubulin: colchicine site, vinca alkaloid site, and a site on the polymerized microtubule to which taxol binds.^{1a,14}

An important basic and essential aspect of this work requires a detailed understanding, on the molecular level, of the "small molecule" binding domain of both the a and β subunits of tubulin. The tertiary structure of the α, β tubulin heterodimer was reported earlier this year by Downing and co-workers at a resolution of 3.7 A using a technique known as electron crystallography¹⁵. This brilliant accomplishment culminates decades of work directed toward the elucidation of this structure and should facilitate the identification of small molecule binding sites, such as the colchicine site, through techniques such as photoaffinity and chemical affinity labelling.

According to one aspect of the present invention therefore there is provided a compound as claimed in claim 1 below.

In some embodiments, R¹ or R³ is a methoxy group, R⁵ is methoxy, R⁶ or R⁷ is OH, and the rest of R¹ to R⁷ are all hydrogen.

In another aspect of the present invention the compound of the invention is for use in inhibiting tubulin polymerization in a tubulin-containing system; the treatment of a host cell afflicted with a neoplastic disease; the treatment of a cancer of the group containing leukaemia, melanoma, and lung, colon, thyroid, CNS, ovarian, renal, prostate and breast cancer; or in selectively targeting and destroying tumour vasculature.

For such uses, R⁵ and R⁵ may both be methoxy, R⁷ may be OH, and the rest of R¹ to R⁷ may all be hydrogen.

Following is a description by way of example only with reference to the accompanying drawings of embodiments of the present invention.
Figure 1 illustrates 3-(3', 4', 5' - trimethoxybenzoyl)-2-(4'-methoxyphenyl)-6-methoxybenzo[*b*]thiophene.
Figure 2 illustrates 2-(3', 4', 5'-trimethoxybenzoyl)-3-(4'-methoxyphenyl)-6-methoxybenzo[b]furan.
Figure 3 illustrates benzo [*b*]thiophene Phenol (BBT-P).
Figure 4 illustrates benzo[*b*]thiophene prodrug (BBT-P).
Figure 5 illustrates *in vivo* biological data for benzo[*b*]thiophene prodrug (BBT-P).
Figure 6 illustrates a synthetic route for preparation of phenylindole derivatives.
Figure 7 illustrates a COSY NMR for 2-phenyl indole (aromatic region) a compound 31.
Figure 8 illustrates a cyclized isomer without aryl migration (no evidence for its formation).
Figure 9 illustrates a preparation of 2-phenylindole 31 in a one-pot reaction.
Figure 10 illustrates a designed synthetic route for preparation of indole-based analogue.
Figure 11 illustrates a preparation of indole-based analogue.
Figure 12 illustrates another synthesis of indole-based disodium prodrug salt
Figure 13 illustrates another synthesis of indole-based disodium prodrug.
Figure 14 illustrates another synthesis of indole-based disodium prodrug.
Figure 15 illustrates a synthesis of indole-based phosphoramidate prodrug.
Figure 16 illustrates another synthesis of indole-based disodium prodrug salt.
Figure 17 illustrates 2-(4'-Methoxyphenyl)-3-(3", 4", 5"-trimethoxybenzoyl)-4-methoxyindole.

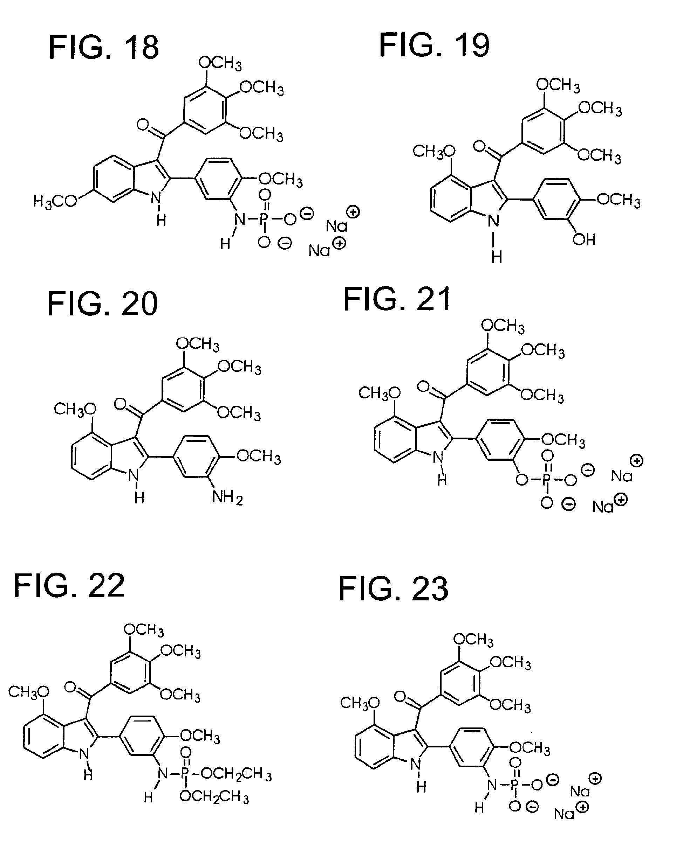

Figure 18 illustrates Disodium2-(3'-phosphoramidate-4'-methoxyphenyl)-3-(3",4",5"-trimethoxybenzoyl)-6-methoxyindole.
Figure 19 illustrates 2-(3'-Hydroxy-4'-methoxyphenyl)-3-(3", 4", 5"-trimethoxybenzoyl)-4-methoxyindole.
Figure 20 illustrates 2-(3'-Amino-4'-methoxyphenyl)-3-(3",4",5"-trimethoxybenzoyl)-4-methoxyindole.
Figure 21. illustrates Disodium 2-[(4'-methoxyphenyl)-3'-O-phosphate]-3-(3",4", 5"trimethoxybenzoyl)-4-methoxyindole.
Figure 22 illustrates 2-(3'-Diethylphosphoramidate-4'-methoxyphenyl)-3-(3",4", 5"-trimethoxybenzoyl)-4-methoxyindole.
Figure 23 illustrates Disodium 2-(3'-phosphoramidate-4'-methoxyphenyl)-3-(3",4", 5"-trimethoxybenzoyl)-4-methoxyindole.

We have developed a working hypothesis suggesting that the discovery of new antimitotic agents may result from the judicious combination of a molecular template (scaffold) which in appropriately substituted form (ie. phenolic moieties, etc.) interacts with estrogen receptor (ER), suitably modified with structural features deemed imperative for tubulin binding (arylalkoxy groups, certain halogen substitutions, etc.). The methoxy aryl functionality seems especially important for increased interaction at the colchicine binding site in certain analogs.¹⁶ Upon formulation of this hypothesis concerning ER molecular templates, our initial design and synthesis efforts centered on benzo [b]thiophene ligands modeled after raloxifene, the selective estrogen receptor modulator (SERM) developed by Eli Lilly and Co.¹⁷ Our initial studies resulted in the preparation of a very active benzo[b]thiophene-based antitubulin agent. ¹⁸⁻²¹ In further support of our hypothesis, recent studies have shown that certain estrogen receptor (ER) binding compounds as structurally modified estradiol congeners (2-methoxyestradiol, for example) interact with tubulin and inhibit tubulin polymerization.²² Estradiol is, of course, perhaps the most important estrogen in humans, and it is intriguing and instructive that the addition of the methoxy aryl motif to this compound makes it interactive with tubulin. It is also noteworthy that 2-methoxyestradiol is a natural mammalian metabolite of estradiol and may play a cell growth regulatory role especially prominent during pregnancy.

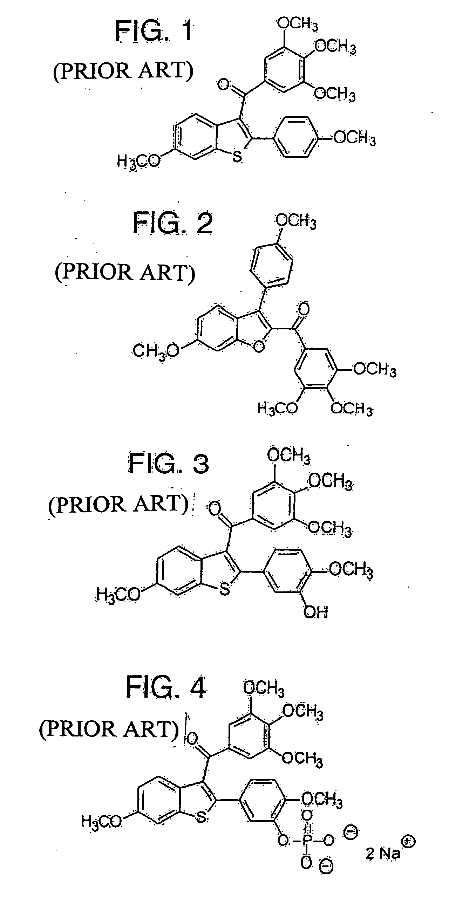

The design premise that molecular skeletons oftraditional estrogen receptor (ER) binding compounds can be modified with structural motifs reminiscent of colchicine and combretastatin A-4 to produce inhibitors oftubulin polymerization has been validated by the benzo[b]thiophene and benzol[b]furan classes of new antimitotic agents.¹⁸⁻²¹ The lead compounds in each series (Figures 1 and 2), demonstrate remarkable biological activity against a variety of human cancer cell lines. For example, the 3,4,5-trimethoxybenzo[b]thiophene (Fig. 1) demonstrates potent cytotoxicity and inhibition of tubulin polymerization. In the NCI 60 cell line panel,²³ this compound produces a mean panel G1₅₀ = 2.63 x 10⁻⁷ M (see Table I).

Inhibition of tubulin polymerization by 3-(3', 4', 5' - trimethoxybenzoyl)-2-(4'-methoxyphenyl)-6-methoxybenzo[b]thiophene. 50% inhibition of the maximum tubulin assembly rate with 1.1 µM drug same assay with conbretastatin A-4 gives a value of 0.73 µM.

Human cancer cell line studies (*in vitro*) by 3-(3', 4', 5' - trimethoxybenzoyl)-2-(4'-methoxyphenyl)-6-methoxybenzo[b]thiophene.

**Table I. Inhibition of tubulin polymerization by 2-(3', 4' 5'-trimethoxybenzoyl)-3-(4'-methoxyphenyl)-6-methoxybenzo[b]furan. IC50 = 2.1 pM (totally flat at 4 pM).**

| Human cancer cell line studies (*in vitro*) by 2-(3', 4', 5'-trimethoxybenzoyl)-3-(4'-methoxyphenyl)-6-methoxybenzo[*b*]furan. | | |
|---|---|---|
| Type of Cancer Cell Line | Cancer Cell Line | GI₅₀ (uglmL) |
| Pancreas - adn | BXPC-3 | 0.038 |
| Neuroblast | SK-N-SH | 0.025 |
| Thyroid ca | SW1736 | 0.047 |
| Lung-NSC | NCI-H460 | 0.041 |
| Pharynx-sqam | FADU | 0.035 |
| Prostate | DU-145 | 0.062 |

In addition, the phenolic derivative of the 3,4-5-trimethoxybenzo[*b*]thiophene compound (Figure 3) has pronounced cytotoxicity and demonstrates outstanding inhibition of tubulin polymerization³⁰ and the pro-drug disodium phosphate salt form of this compound (Figure 4) demonstrates *in vitro* and *in vivo* cytotoxicity as a vascular targeting and destruction agent (which includes a component of tubulin binding (phenolic form of drug)^{30,31} and subsequent inhibition of tubulin polymerization).

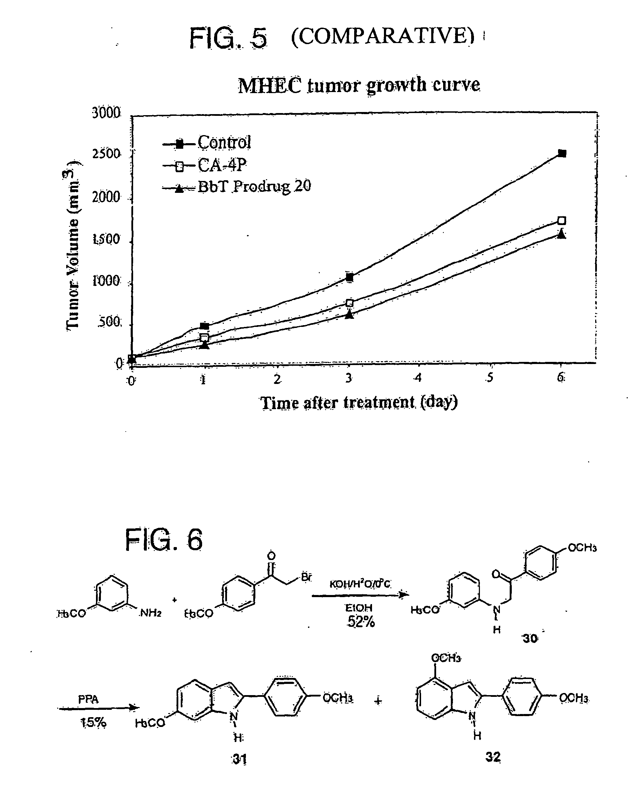

Initial *in vivo* studies are very encouraging (see Figure 5). Female scid mice were single dose ip administered with CA-4P, and benzo[*b*]thiophene phosphate prodrug at 400mg/kg (i.e. MDT of CA-4P) after one week of MHEC inoculation (1 x 10⁻⁶/mouse). Studies were carried out through a collaboration with Professors Ronald W. Pero and Klaus Edvardsen, University of Lund, Sweden (Note: PbT Prodrug 20 is the same compound that is referred to as BBT-P).

Based on these promising research results, our interest in designing an indole based antimitotic agent was initiated, and a synthetic route was designed according to the synthesis of the benzo[*b*]thiophene derivatives.

The possibility clearly exists that some of the new indole-based ligands described herein, which are structurally related to combretastatin A-4, may also function through additional biological mechanisms involving anti-angiogenic activity. Clearly the ability to selectively disrupt the blood-flow to developing tumor cells is a potential breakthrough in the ever up-hill battle against cancer. Certain phenylindoles have been noted for inhibiting tubulin polymerization.²⁶

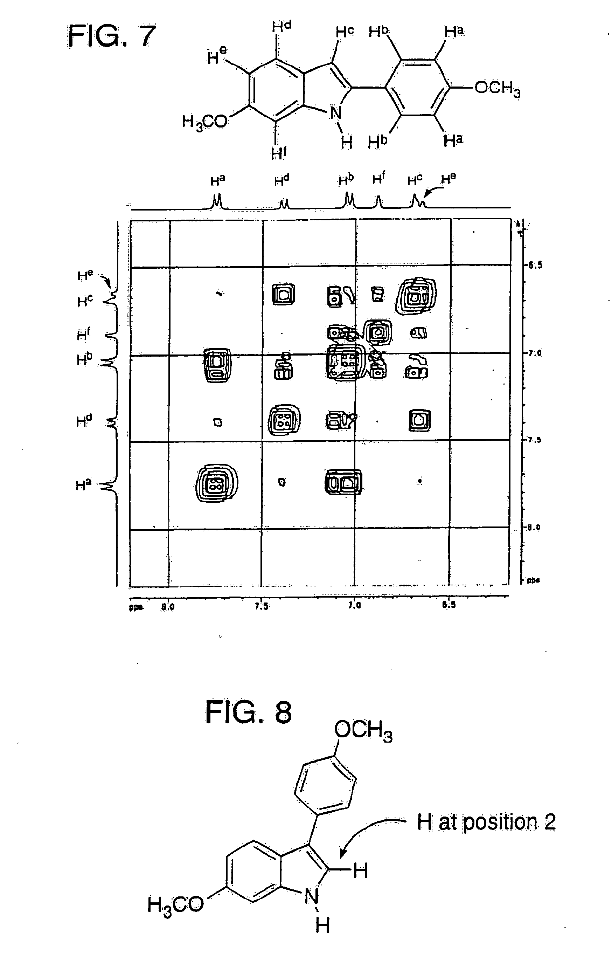

A typical synthesis of indole-based ligand 33 is shown in Figures 6, 9, and 11. Secondary amine 30 was prepared by treatment of *m*-anisidine and 2-bromo-4methoxyacetophenone under basic condition (ethanolic potassium hydroxide) at 0°C. Treatment of amine 30 with PPA resulted in the formation of two regioisomers. These isomers have poor solubility in EtOAc, CH₂Cl₂ and EtOH. Indole 31 was purified (from indole 32) by trituration in acetone. The structure of this isomer was confirmed by NMR analysis. COSY NMR was taken in order to study, in detail, the coupling relationship between the protons. The enlarged COSY spectrum for the aromatic region of ligand 31 is shown in Figure 7. This COSY NMR spectrum, shows a strong coupling between H^{a} and H^{b} which each appear as a doublet. H^{c} is coupled by the proton attached to the nitrogen into a small doublet. H^{d} is coupled only by H^{e} into a corresponding doublet, while H^{e} is coupled both by an ortho coupling (H^{d}) and by a meta coupling (H^{f}) into a doublet of doublet pattern. H^{f} is coupled by H^{e} into a doublet. Further evidence of the formation of2-phenyl indole 31 is the chemical shift of the proton H^{c} on the ring which contains nitrogen. Though computer modeling (ChemDraw Ultra 4.5), the theoretical chemical shift value of 6.4 ppm is predicted for proton H^{c} (at the 3 position), which matches the peak shown in the actual NMR spectrum at 6.6 ppm. For the case where the proton is at the 2 position (Figure 8), the chemical shift is predicted to be 7.03 ppm, which does not match any peak in the spectrum that was obtained. Based collectively on these studies, the formation of isomer 31 is confirmed, and the migration of the methoxyphenyl system is evidenced. The other isomer (indole 32) is soluble in acetone and is much more difficult to obtain in pure form (see Figure 6).

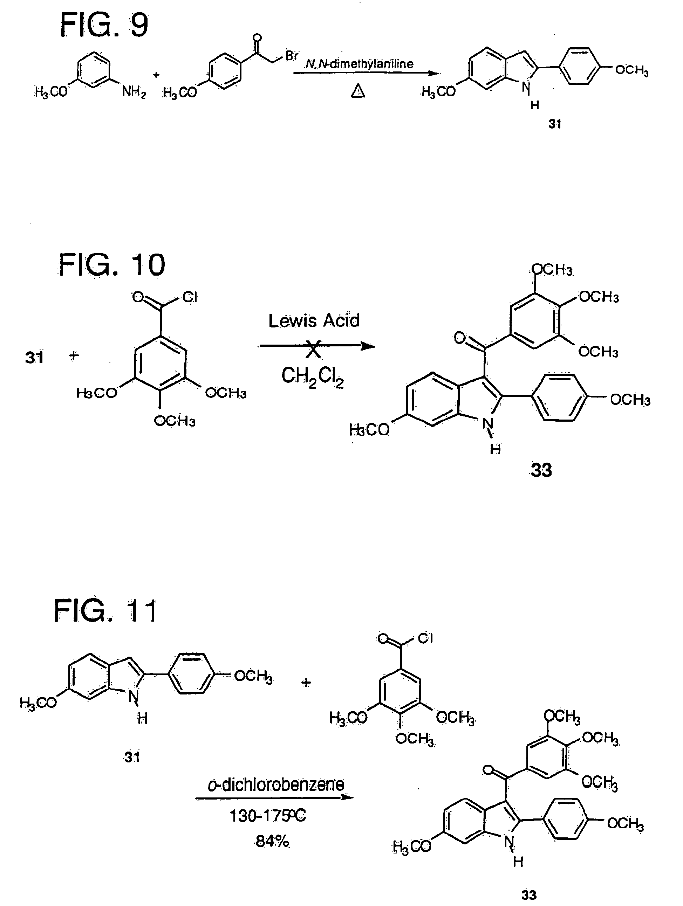

Alternatively, another synthetic methodology can also be applied to the preparation of the desired 2-phenylindole. In 1984, Angerer and co-workers reported the synthesis of 2-phenylindoles in a one-pot reaction sequence (Figure 9) as a route toward the development of new therapeutic agents for the treatment of endocrine disorders.²⁴

Following this procedure (Figure 9), two arylindole regioisomers were obtained in good yield. Recrystallization in EtOH afforded the desired isomer, 2-phenylindole 31, as a white crystalline material.

In order to synthesize the indole-based analog 33, Friedel-Crafts acylation was carried out by treating indole 31 with 3,4,5-trimethoxybenzoyl chloride in the presence of the Lewis-Acid AlCl₃ (Figure 10). The reaction did not work under the regular conditions and only starting material was obtained following work-up. Attempts to modify the reaction conditions by increasing the reaction temperature or using other Lewis Acids, such as TiCl₄, proved futile as well. Starting material was recovered in all cases. One possible explanation for this result is the fact that the nitrogen atom (containing a lone pair of electrons and an acidic proton) may disrupt the acylation process. According to this analysis, a Grignard reagent (ethylmagnesium bromide) was used to protect this nitrogen prior to the Friedel-Crafis acylation step. Still, only starting material was obtained following the reaction. Therefore, a new synthetic approach was brought into this study.

In 1977, Inion and co-workers reported the synthesis of a variety of aminoalkoxy4-benzoyl-3-indoles.²⁵ The benzoate indole product was prepared by treatment of indole with the appropriate benzoyl chloride with heating (130-150°C). HCl is generated under these conditions. A similar synthetic approach was used in the synthesis of the desired trimethoxybenzoate indole ligand 33 (Figure 11).

The precursor, indole 31, was mixed with trimethoxybenzoyl chloride. Since both reagents are solid, a solvent with a high boiling point was needed. 1,2-dichlorobenzene was chosen in this case since it has a boiling point of 1 80°C. Under these condition, indole 33 was obtained in moderate yield following purification by flash column chromatography and recrystallization. NMR spectroscopy suggests that the structure of indole 33 is that indicated in Figure 11.

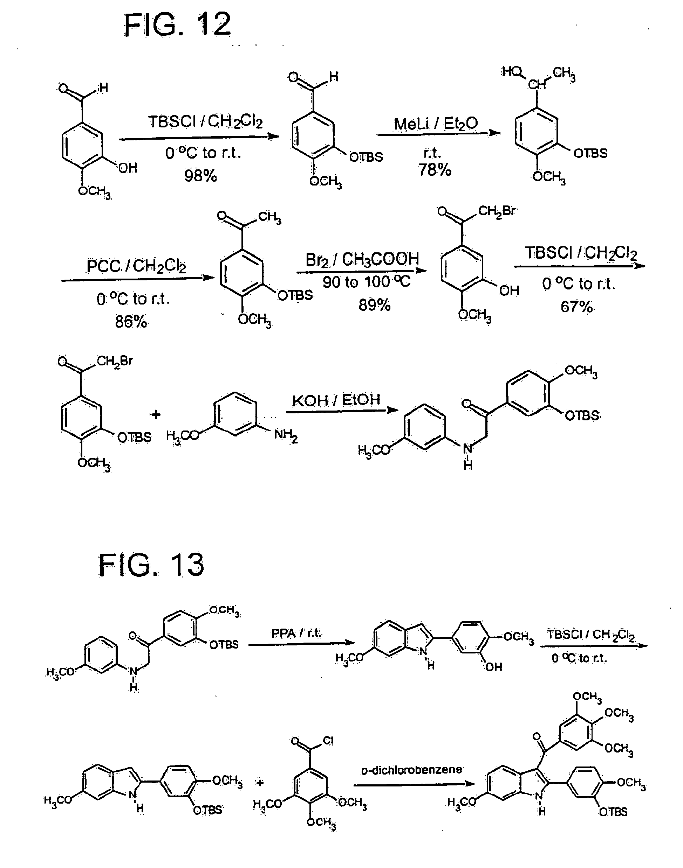

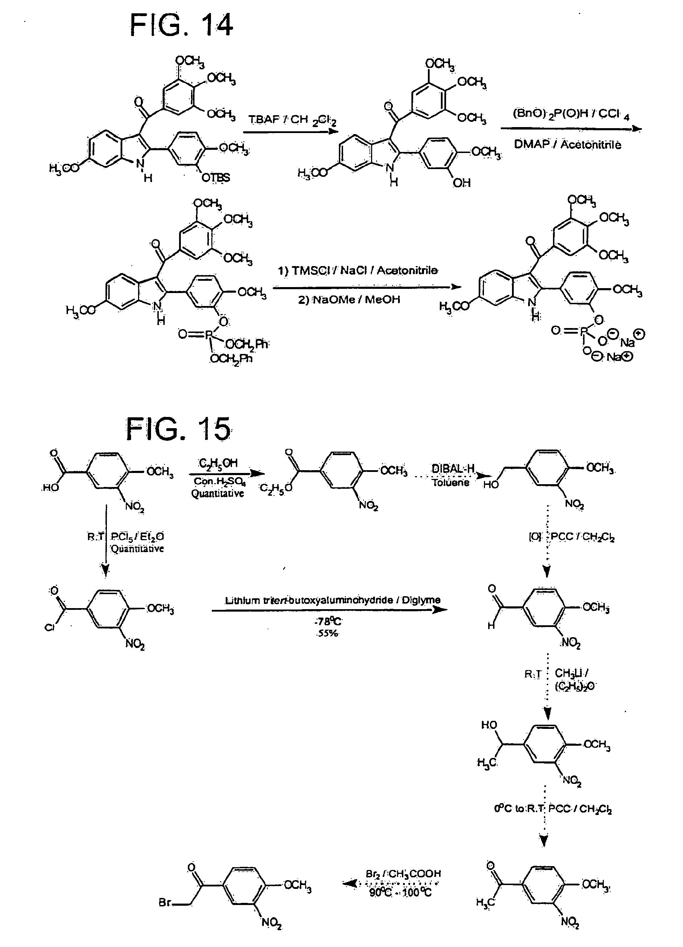

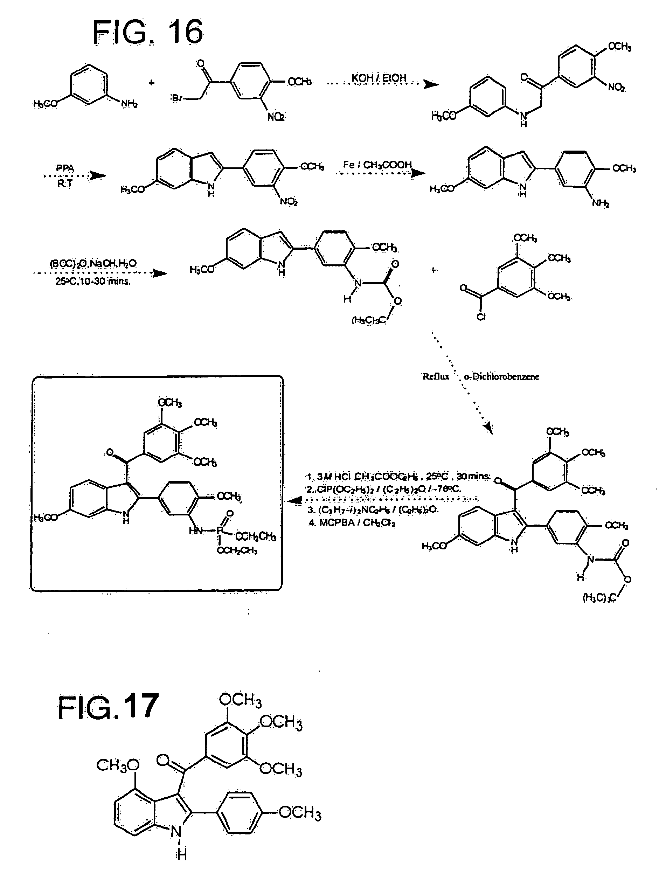

Based on promising results obtained with benzo[b]thiophene and benzofuran analogs, the preparation of phosphate salts is detailed in Figures 12-14 and the preparation of analogs is detailed in Figures 15-16.

### Anti-Angiogenesis

The growth of a tumor depends on the generation of blood vessels which will provide all the metabolites required during cell division. The development of anti-angiogenic compounds is especially useful in the treatment of solid tumors, since these compounds have the potential capability of selectively disrupting the vasculature of tumor cells while leaving healthy cells in a viable situation. The combretastatin A-4 prodrug has demonstrated anti-angiogenic activity since small doses of the drug are toxic to tumor vasculature.²⁸ Enhanced cytotoxic activity was observed against endothelial cells associated with the tumor vasculature of cancerous cells, while at the same time it was reported to have no effect against other endothelial cells which are located distant from the tumor itself. ^{28,29} The mechanism of action of combretastatin A-4 prodrug, as an anti-angiogenic drug for cancer treatment, is under investigation because the development of blood vessels is crucial for the survival and growth of solid tumors. One proposed mechanism for anti-angiogenesis involves induction of apoptosis (cell suicide) of the cells instead of necrosis. An evaluation of the ability of the new phosphoramidate, along with structurally similar compounds, to induce apoptosis of endothelial cells will be undertaken in the near future.

### Example 1

### SYNTHESIS OF THE INDOLE-BASED ANTI-TUBULIN AGENTS

### Preparation of 2-Phenyl Indole 31

### Method I (2 steps):

To a well-stirred solution of KOH (0.926 g, 16.5 mmol) in EtOH (18 ml) and H₂0 (9 ml) at rt was added *m*-anisidine (2.192 g, 17.84 mmol) by syringe. The solution was then stirred at 0°C. After 10 min, the solution of 2-bromo-4-methoxyacetophenone (4.09 g, 17.80 mmol) was added dropwise with an addition funnel over a 40 minute period. After 24 h, 0°C to rt, water was added. The product was isolated by extraction (1 H HCl, NaHCO3, brine, MgSO4). The product was purified by recrystallization (50:50 EtOAc:hexanes) to afford secondary amine 30 (2.46 g, 9.07 mmol, 52%) as yellow solid.

¹H NMR (CDCl₃): δ 7.98 (2H, D, *J* = 8.9 Hz), 7.12 (1H, t, *J* 8.1 Hz), 6.97 (2H, d, *J* 8.9 Hz), 6.30 (3H, m), 4.54 (2H, s), 3.88 (3H, s), 3.79 (3H, s).

Polyphosphoric acid (PPA) was charged to a round-bottom flask and the temperature was raised to 80°C with vigirous stirring. To this flask was added the foregoing amine 30 (4.0 g, 14.74 mmol) in 6 portions over a 30 minute period. After 2 h, 80°C to 90°C, water was added. The product was isolated by extraction (EtOAc, NaHCO₃, brine, MgSO₄). Purification by recrystallization (acetone) afforded indole 31 (0.544 g, 2.15 mmol, 15%) as a pale yellow solid.

¹H NMR (CDCl₃): δ 11.24 (1H, br, s), 7.72 (2H, d, *J* 8.82 Hz), 7.36 (1H, d, *J* = 8.57 Hz), 7.00 (2H, d, *J* = 8.84 Hz), 6.85 (1H, d, *J* = 2.07 Hz), 6.66 (1H, d, *J* = 1.66 Hz), 6.63 (1H, dd, *J* 8.59, 2.28 Hz), 3.78 (3H, s), 3.77 (3H, s).

¹³C NMR (CDCl₃): δ 158.15, 155.22, 137.44, 136.33, 125.60, 124.93, 122.82, 120.04, 114.07, 109.00, 96.97, 94.01, 54.93, 54.88.

### Method 2(1 step):

To a boiling mixture of in-anisidine (1.56 ml, 20.0 minol) and *N*,*N*-dimethylaniline (3.5 ml) was added 2-bromo-4-methoxyacetophenone (1.37 g in EtOAc, 6.00 mmol) slowly by syringe. After addition, the mixture was kept at 170 ° C for 1 hour. The reaction mixture was cooled to room temperature and a dark colored solid was formed. EtOAc was added along with HCl (2 N). The aqueous layer was extracted with EtOAc several times. The combined organic layers were washed with brine, and dried over MgSO₄. Solvent was removed under the reduced pressure to afford a dark brown colored solid. Purification by recrystallization in EtOH afforded indole 31 as a white crystalline material.

¹H NMR(CDCl₃): δ 11.24 (1H,br,s),7.72(2H,d, *J* 8.82 Hz), 7.36 (IH,d, *J* 8.57 Hz),7.00 (2H,d, *J* = 8.84 Hz), 6.85 (IH, d, *J*= 2.07 Hz), 6.66 (IH, d, *J*= 1.66 Hz), 6.63 (IH, dd, *J* 8.59, 2.28 Hz),3.78 (3H, s), 3.77 (3H, s).

¹³C NMR (CDCl₃): δ 158.15, 155.22, 137.44, 136.33, 125.60, 124.93, 122.82, 120.04, 114.07, 109.00, 96.97, 94.01, 54.93, 54.88.

Melting Point: 208-229.5 °C

HRMS (El) M+ calcd for CH₁₆N0₂ 253.3035, found 253.1060.

### Preparation of Trimethoxybenzoate 2-Phenylindole 33

To a well stirred solution of indole 31(0.502 g, 1.98 mmol) in o.dichlorobenzene (10 ml) was added trimethoxybenzoylchloride (0.692 g, 3.00 mmol). The reaction mixture was heated to reflux for 12 hours. Solvent was removed by distillation under reduced pressure. After cooling down to room temperature, a dark solid formed which was dissolved in chloroform and purified by silica gel column chromatography with chloroform as the eluent. The collected mixture was again purified by column chromatography (50:50 hexanes:EtOAc) affording trimethoxybenzyl indole 33 (0.744 g, 1.66 mmol, 84%) as a yellow oily gel. Pale yellow-green crystals were obtained by recrystallization from a mixture of ethanol and hexanes.

¹H NMR (CDCl₃): δ 8.63 (1H, br, s), 7.88 (1H, d, *J* = 9.39 Hz), 7.24 (2H, d, *J*= 8.78 Hz), 6.95(2H, s), 6.90 (2H, m), 6.71 (2H, d, *J*= 8.79 Hz), 3.86 (3H, s), 3.80 (3H, s), 3.73 (3H, s), 3.68 (6H, s);

¹³C NMR (CDCl₃): δ 192.23, 159.73, 157.06, 152.42, 142.85, 141.01, 136.41, 134.65, 130.16, 124.28, 122.94, 122.17, 113.67, 112.46, 111.52, 107.24, 94.54, 60.78, 55.92, 55.54, 55.14.

Melting Point: 153-155 °C

Anal. Calcd for C₂₆H₂₅NO₆: C, 69.79; H, 5.63; H, 3.13. Found: C, 69.61; H, 5.63; N, 3.01.

### EXAMPLE 2

### INHIBITION OF TUBULIN POLYMERIZATION ASSAY

IC₅₀ values for tubulin polymerization were determined according to the procedure described in Bai et al. Purified tubulin is obtained from bovine brain cells as described in Hamel and Lin. Various amounts of inhibitor were preincubated for 15 minutes at 37 °C with purified tubulin. After the incubation period, the reaction was cooled and GTP was added to induce tubulin polymerization. Polymerization was then monitored in a Gilford spectrophotometer at 350 nm. The final reaction mixtures (0.25 ml) contained 1.5 mg/ml tubulin, 0.6 mg/ml microtubule-associated proteins (MAPs), 0.5 mM GTP, 0.5 mlM MgCl₂, 4% DMSO and 0.1M 4-morpholineethanesulfonate buffer (MES, pH 6.4). IC₅₀ is the amount of inhibitor needed to inhibit tubulin polymerization 50% with respect to the amount of inhibition that occurs in the absence of inhibitor. The IC₅₀ value determined for 3 -(3' ,4' ,5' -trimethoxybenzoyl)-2-(4' -methoxyphenyl)-6-methoxyindole was 0.5-1.5 µM.

### EXAMPLE 3

### CYTOTOXIC ASSAY WITH P388 LEUKEMIA CELLS

One of the newly prepared compounds was evaluated for cytotoxic activity against P388 leukemia cells using an assay system similar to the National Cancer Institute procedure described below and in Monks et al. The ED50 value (defined as the effective dosage required to inhibit 50% of cell growth) of 3-(3',4',5' trimethoxybenzoyl)-2-(4'-methoxyphenyl)-6-methoxyindole was found to be 0.0133 µg/mL.

### EXAMPLE 4

### GROWTH INHIBITORY ACTIVITY AGAINST OTHER

### CANCER CELL LINES

3 -(3' ,4' ,5'-Trimethoxybenzoyl)-2-(4'-methoxyphenyl)-6-methoxyindole was evaluated in terms of growth inhibitory activity against several human cancer cell lines, including pancreas, ovarian, CNS, lung-NSC, colon, and prostate lines. The assay used is described in Monks et al. Briefly, the cell suspensions, diluted according to the particular cell type and the expected target cell density (5,000-40,000 cells per well based on cell growth characteristics), were added by pipet (100 µl) to 96-well microtiter plates. Inoculates were allowed a preincubation time of 24-28 hours at 37°C for stabilization. Incubation with the inhibitor compounds lasted for 48 hours in 5% CO₂ atmosphere and 100% humidity. Determination of cell growth was done by in situ fixation of cells, followed by staining with a protein-binding dye, sulforhodamine B (SRB), which binds to the basic amino acids of cellular macromolecules. The solubilized stain was measured spectrophotometrically. The results of these assays are shown in Table 1. Gl₅₀ is defined as the dosage required to inhibit tumor cell growth by 50%.

**Table II. Activity of Indole Ligand Against Selected Human Cancer Cell lines (In Vitro).**

| Indole-based Ligand 33 | | |
|---|---|---|
| CELL TYPE | CELL LINE | GI₅₀ (µG/mL) |
| Pancreas-a | BXPC-3 | 2.0 x 10⁻³ |
| Ovarian | OVCAR-3 | 2.4 x 10⁻³ |
| CNS | SF-295 | 2.4 x 10⁻³ |
| Lung-NSC | NCI-H460 | 2.6 x 10⁻³ |
| Colon | KM20L2 | 1.7 x 10⁻³ |
| Prostate | DU-145 | 2.3 x 10⁻³ |

Indole and indole containing compounds of therapeutic efficacy have been known for many, many years. What is truly unique about the indole compounds described in this application is the fact that these compounds are the first (to the best of our knowledge) indole-based ligands to incorporate the 3,4,5-trimethoxyaryl motif reminiscent of colchicine and combretastatin A-4 arranged in an appropriate molecular conformation such that a pseudo aryl-aryl pi stacking interaction can take place. It is our contention that such an aryl-aryl interaction of the appropriate centroid-to-centroid distance (approximately 4.7 Å) is imperative for enhanced binding affinity to the colchicine site on β-tubulin. It is this binding that ultimately leads to an inhibition of tubulin polymerization which manifests itself as a cytotoxic event. It should be readily apparent to any practitioner skilled in the art that there are various ways of appending trimethoxyaryl and trimethoxyaroyl groups around an indole molecular scaffold in a manner which will result in a similar molecular conformation capable of undergoing pseudo pi-pi stacking. In addition, although the trimethoxyaryl motif seems optimal for enhanced tubulin binding, it is also very possible that another combination of alkoxy substituents (such as ethoxy, propoxy, isopropoxy, allyloxy, etc.) either as a trisubstituted pattern or as disubstituted (with one type of alkoxy moiety) and monosubstituted (with a different alkoxy moiety), or with three distinct types of alkoxy moieties may also have good tubulin binding characteristics. It is also conceivable that instead of having aryl alkoxy groups, it may be possible to substitute simply aryl-alkyl and aryl-alkenyl moieties and still maintaln the enhanced cytotoxicity profile. Phenolic groups may also have activity on these described indole ligands. The synthesis of any of these modified indole-ligands will be very straight-forward for anyone skilled in the art, and often will only involve a different choice of initial starting materials. To prepare these alternative ligands, the same synthetic schemes (Figures 6, 9, 11, 12-16), or similar schemes with only slight modifications may be employed. In previous studies with the benzo[*b*]thiophene ligands, we have demonstrated that the carbonyl group can be replaced with an oxygen to generate a new compound which maintains the same or similar biological efficacy with tubulin. Similarly, the replacement of the carbonyl group in the described indole ligand may be replaced with an oxygen atom (ether linkage) to generate a new derivative which would be predicted to have good activity with tubulin. This compound may be prepared by an addition elimination reaction utilizing the trimethoxyphenolic anion as a nucleophile as described by us for the benzo[*b*]thiophene compounds. Other linkage atoms between the aryl aryl rings are conceivable as well.

### Literature Cited

1)
   (a) For a recent review of numerous antitubulin and antimitotic agents see: Hamel, E., Antimitotic Natural Products and Their Interactions with Tubulin, Medicinal Research Reviews, 1996, 16, 207.
   (b) Gerwick, W. H.; Proteau, P. J.; Nagle, D. G.; Hamel, B.; Blokhin, A.; Slate, D. L., Structure of Curacin A, a Novel Antimitotic, Antiproliferative, and Brine Shrimp Toxic Natural Product from the Marine Cyanobacterium Lyngbya majuscula, J. Org. Chem. 1994, 59, 1243.
   (c) Gianna Kakou, P.; Sackett, D.; Fojo, T.; Tubulin/Microtubes: Still a promising Target for New Chemotherapeutic Agents, J. Natl Cancer Inst., 2000, 92, 182.
2) Owellen, R. J.; Hartke, C. A.; Kickerson, R. M.; Hams, F. 0., Inhibition of Tubulin-Microtubule Polymerization by Drugs of the Vinca Alkaloid Class, Cancer Res. 1976, 36, 1499.
3) Lavielle, G.; Havtefaye, P.; Schaeffer, C.; Boutin, J. A.; Cudennec, C. A.; Pierre, A., New α-Amino Phosphonic Acid Derivatives of Vinblastine: Chemistry and Antitumor Activity, J Med Chem. 1991, 34, 1998.
4)
   (a) Kingston, D. G. I.; Samaranayake, G.; Ivey, C. A., The Chemistry of Taxol, a Clinically Useful Anticancer Agent, J. Nat. Prod. 1990, 53, 1.
   (b) Schiff, P. B.; Fant, J.; Horwitz, S. B., Promotion of Microtubule Assembly In Vitro by Taxol, Nature, 1979, 277, 665.
   (c) Swindell, C. S.; Krauss, N. B.; Horwitz, S. B.; Ringel, I., Biologically Active Taxol Analogs with Deleted A-ring Side Chain Substituents and Variable C-2Æ Configurations, J. Med Chem. 1991, 34, 1176.
   (d) Pamess, J.; Horwitz, S. B., Taxol Binds to Polymerized Tubulin In Vitro, J. Cell Biol. 1981, 91, 479.
5)
   (a) Nakada, M.; Kobayashi, S.; Iwasaki, S.; Ohno, M., The First Total Synthesis of the Antitumor Macrolide Rhizoxin: Synthesis of the Key Building Blocks, Tetrahedron Lett. 1993, 34, 1035.
   (b) Nakada, M.; Kobayashi, S.; Iwasaki, S.; Ohno, M., The First Total Synthesis of the Antitumor Macrolide Rhizoxin,Tetrahedron Lett. 1993, 34, 1039.
   (c) Boger, D.L.; Curran, T. T., Synthesis of the Lower Subunit of Rhizoxin, J. Org. Chem. 1992, 57, 2235.
   (d) Rao, A. V. R.; Sharma, G. V. M.; Bhanu, M. N., Radical Mediated Enantioselective Construction of C-1 to C-9 Segment of Rhizoxin, Tetrahedron Lett. 1992, 33, 3907.
   (e) Kobayashi, S.; Nakada, M.; Ohno, M., Synthetic Study on an Antitumor Antibiotic Rhizoxin by Using an Enzymatic Process on Prochiral betaSubstituted Glutarates, Pure Appl. Chem. 1992, 64, 1121*.*
   (f) Kobayashi, S.; Nakada, M.; Ohno, M., Synthetic Study on an Antitumor Antibiotic Rhizoxin by Using an Enzymatic Process on Prochiral betaSubstituted Glutarates Indian J. Chem., Sect. B. 1993, 32B, 159.
   (g) Rao, A. V. R.; Bhanu, M. N.; Sharma, G. V. M., Studies Directed Towards the Total Synthesis of Rhizoxin: Stereoselective Synthesis of C- 12 to C- 18 Segment, Tetrahedron Lett. 1993, 34, 707.
6)
   (a) Lin, C. M.; Ho, H. H.; Pettit, G. R.; Hamel, E., Antimitotic Natural Products Combretastatin A-4 and Combretastatin A-2: Studies on the Mechanism of Their Inhibition of the Binding of Colchicine to Tubulin, Biochemistry 1989, 28, 6984.
   (b) Pettit, G. R.; Cragg, G. M.; Singh, S. B., Antineoplastic agents, 122. Constituents of Combretum caffrum, J. Nat. Prod 1987, 50, 386.
   (c) Pettit, G. R.; Singh, S. B.; Cragg, G. M., Synthesis of Natural (-)Combretastatin, J. Org. Chem. 1985, 50, 3404.
   (d) Pettit, G. R.; Cragg, G. M.; Herald, D. L.; Schmidt, J. M.; Lohavanijaya, P., Isolation and Structure of combretastatin, Can. J Chem. 1982, 60, 1374.
   (e) Dorr, R. T.; Dvorakova, K.; Snead, K.; Alberts, D. S.; Salmon, S. E.; Pettit, G. R., Antitumor Activity of Combretastatin A4 Phosphate, a Natural Product Tubulin Inhibitor, Invest. New Drugs, 1996, 14, 131.
7)
   (a) Hammonds, T. R.; Denyer, S. P.; Jackson, D. B.; Irving, W. L., Studies To Show That With Podophyllotoxin the Early Replicative Stages of Herpes Simplex Virus Type 1 Depend Upon Functional Cytoplasmic Microtubules, J. Med. Microbiol., 1996, 45, 167.
   (b) Cortese, F.; Bhattacharyya, B.; Wolff, J., Podophyllotoxin as a Probe for the Colchicine Binding Site of Tubulin, J. Biol. Chem., 1977, 252, 1134.
8) Nicolaou, K. C., Winssinger, N., Pastor, J., Ninkovic, S., Sarabia, F., He, Y., Vourloumis, D., Yang, Z., Oi, T., Giannakakou, P., Hamel, B., Sythesis of Epothilones A and B in Solid and Solution Phase, Nature, 1997, 387, 268-272.
9)
   (a) Pettit, G. R., Kamano, Y., Herald, C. L., Tuinman, A. A., Boettner, F. E., Kizu, H., Schmidt, J. M., Baczynskyj, L., Tomer, K. B., Bontems, R. J., The Isolation and Structure of a Remarkable Marine Animal Antineoplastic Contituent: Dolastatin 10, J. Am. Chem. Soc., 1987, 109, 6883-6885.
   (b) Pettit, G. R., Srirangam, J. K., Barkoczy, J., Williams, M. D., Boyd, M. R., Hamel, E., Pettit, R. K., Hogan F., Bai, R., Chapuis, J. C., McAllister, S. C., Schmidt, J. M., Antineoplastic Agents 365: Dolastatin 10 SAR Probes, Anti-Cancer Drug Des., 1998, 13, 243-277.
10) Zhang, X.; Smith, C. D., Microtubule Effects of Welwistatin, a Cyanobacterial Indolinone that Circumvents Multiple Drug Resistance, Molecular Pharmacology, 1996, 49,288.
11) Pettit, G. R., Told, B., Herald, D. L., Verdier-Pinard, P., Boyd, M. R., Hamel, E., Pettit, R. K., Antineoplastic Agents 379. Synthesis of Phenstatin Phosphate, J. Med Chem., 1998, 41, 1688-1695.
12) Jiang, J. B.; Hesson, D. P.; Dusak, B. A.; Dexter, D. L.; Kang, G. J.; Hamel, B.; Synthesis and Biological Evaluation of 2-Styrylquinazolin-4(3H)-ones, a New Class of Antimitotic Anticancer Agents Which Inhibit Tubulin Polymerization, J. Med. Chem. 1990, 33, 1721.
13) Cushman, M.; Nagarathnam, D.; Gopal, D.; Chakraborti, A. K.; Lin, C. M.; Hamel, E. Synthesis and Evaluation of Stilbene and Dihydrostilbene Derivatives as Potential Anticancer Agents That Inhibit Tubulin Polymerization, J. Med Chem. 1991, 34, 2579.
14)
   (a) Sawada, T.; Kato, Y.; Kobayashi, H.; Hashimoto, Y.; Watanabe, T.; Sugiyama, Y.; Iwasaki, S., A Fluorescent Probe and a Photoaffinity Labeling Reagent to Study the Binding Site of Maytansine and Rhizoxin on Tubulin, Bioconjugate Chem., 1993, 4, 284.
   (b) Rao, S.; Horwitz, S. B.; Ringel, I., Direct Photoaffinity Labeling of Tubulin with Taxol, J. Natl. Cancer Inst., 1992, 84, 785.
   (c) Chavan, A. J.; Richardson, S. K.; Kim, H.; Haley, B. E.; Watt, D. S., Forskolin Photoaffinity Probes for the Evaluation of Tubulin Binding Sites, Bioconjugate Chem. 1993, 4, 268.
   (d) Sawada, T.; Kobayashi, H.; Hashimoto, Y.; Iwasaki, S., Identification of the Fragment Photoaffinity-labeled with Azidodansyl-rhizoxin as Met-363 - Lys-379 on beta-Tubulin, Biochem. Pharmacol. 1993, 45, 1387.
   (e) Staretz, M. E.; Hastie, S. B., Synthesis, Photochemical Reactions, and Tubulin Binding of Novel Photoaffinity Labeling Derivatives of Coichicine, J. Org. Chem. 1993, 58, 1589.
   (f) Hahn, K. M.; Hastie, S. B.; Sundberg, R. J., Synthesis and Evaluation of 2-Diazo-3 ,3 ,3 -trifluoropropanoyl Derivatives of Colchicine and Podophyllotoxin as Photoaffinity Labels: Reactivity, Photochemistry, and Tubulin Binding, Photochem. Photobiol. 1992, 55, 17.
   (g) Sawada, T.; Hashimoto, Y.; Li, Y.; Kobayashi, H.; Iwasaki, S., Fluorescent and Photoaffinity Labeling Derivatives of Rhizoxin, Biochem. Biophys. Res. Commun. 1991, 178, 558.
   (h) Wolff, J.; j(nipling. L.; Cahnmann, H. J.; Palumbo, G., Direct Photoaffmity Labeling of Tubulin with Colchicine, Proc. Natl. Acad Sci. US.A. 1991, 88, 2820.
   (i) Floyd. L. J.; Bames, L. D.; Williams, R. F., Photoaffinity Labeling of Tubulin with (2-Nitro-4-azidophenyl)deacetylcolchicine: Direct Evidence for Two Colchicine Binding Sites, Biochemistry, 1989, 28, 8515.
   (j) Safa, A. R.; Hamel, E.; Felsted, R. L., Photoaffinity Labeling of Tubulin Subunits with a Photoactive Analog of Vinblastine, Biochemistry 1987, 26, 97.
   (k) Williams, R. F.; Mumford, C. L.; Williams, G. A.; Floyd, L. J.; Aivaliotis, M. J.; Martinez, R. A.; Robinson, A. K.; Bames, L. D., A Photoaffinity Derivative of Colchicine: 6-(4'-Azido-2'-nitrophenylamino)hexanoyldeacetylcolchicine. Photolabeling and Location of the Colchicine-binding Site on the alpha-subunit of Tubulin, J Biol. Chem. 1985, 260, 13794.
15) Nogales, E., Wolf, S. G., and Downing, K. H., Structure fo the ct,~ Tubulin Dimer by Electron Crystallography, Nature, 1998, 391, 199-203.
16) Shirai, R.; Tokuda, K.; Koiso, Y.; Iwasaki, S., Synthesis and AntiTubulin Activity of Aza-Combretastatins, Biomedical Chem. Lett. 1994, 699.
17)
   (a) Jones, C. K.; Jevnikar, M. G.; Pike, A. J.; Peters, M. K.; Black, L. J.; Thompson, A. R.; Falcone, J. F.; Clemens, J. A., Antiestrogens. 2. Structure-Activity Studies in a Series of 3-Aroyl-2-arylbenzo[b]thiophene Derivatives Leading to [6-Hydroxy-2-(4-hydroxyphenyl)benzo[b]thien-3-yl][4-[2-(1-piperidinyl) ethoxy] phenylimethanone Hydrochioride (LY156758), a Remarkably Effective Estrogen Antagonist with Only Minimal Intrinsic Estrogenicity, J. Med Chem. 1984, 27, 1057.
   (b) Grese, T. A.; Cho, S.; Finley, D. R.; Godfrey, A. G.; Jones, C. D.; Lugar III, C. W.; Martin, M. J.; Matsumoto, K.; Pennington, L. D.; Winter, M. A.; Adrian, M. D.; Cole, H. W.; Magee, D. E.; Phillips, D. L.; Rowley, E. R.; Short, L.; Glasebrook, A. L.; Bryant, H. R., Structure-Activity Relationships of Selective Estrogen Receptor Modulators: Modifications to the 2-Arylbenzothiophene Core of Raloxifene, J Med Chem., 1997, 40, 146.
   (c) Palkowitz, A. D.; Glasebrook, A. L.; Thrasher, K. J.; Hauser, K. L.; Short, L. L.; Phillips, D. L.; Muehl, B. S.; Sato, M.; Shetler, P. K.; Cullinan, G. J.; Pell, T. R.; Bryant, H. U., Discovery and Synthesis of [6-Hydroxy-3-[4-[2-(1-piperidinyl)ethoxy]phenoxy]-2-(4-hydroxyphenyl)]benzo[b]thiophene: A Novel, Highly Potent, Selective Estrogen Receptor Modulator, J. Med Chem., 1997, 40, 1407.
18) Pinney, K.G., Anti-Mitotic Agents Which Inhibit Tubulin Polymerization, Baylor University, Application for United States Letters Patent, Filed, March 6, 1997. Patent Number: 5,886,025. Issued, March 23, 1999.
19) Pinney, K. G.; Mejia, P.; Mocharla, V. P.; Shirlai, A.; Pettit, G. R., Anti-Mitotic Agents Which Inhibit Tubulin Polymerization, PCT Application Pending, filed under the Patent Cooperation Treaty on March 6, 1998 and designating all PCT member states. Filed jointly by Baylor University, Arizona Disease Control Research Commission, and Arizona State University.
20) Mullica, D. F.; Pinney, K. G.; Mocharla, V. P.; Dingeman, K. M.; Bounds, A. D.; Sappenfield, E. L., Characterization and Structural Analyses of Trimethoxy and Triethoxybenzo[b]thiophene, J. Chem. Cryst., 1998, 28, 289-295.
21) Pmnney, K. G.; Dingeman, K. D.; Bounds, A. D.; Mocharla, V. P.; Pettit, G. R.; Bai, R.; Hamel, E., A New Anti-Tubulin Agent Containing the Benzo[b]thiophene Ring System, Bioorganic and Medicinal Chemistry Letters, 1999, 9, 1081-1086.
22)
   (a) D'Amato, R. J.; Lin, C. M.; Flynn, E.; Folkman, J.; Hamel, E., 2Methoxyestradiol, an endogenous mamalian metabolite, inhibits tubulin polymerization by interacting at the colchicine site, Proc. Natl. Acad Sci. 1994, 91, 3964.
   (b) Cushman, M.; He, H-M.; Katzenellenbogen, J. A.; Lin, C. M.; Hamel, E., Synthesis, Antitubulin and Antimitotic Activity, and Cytotoxicity of Analogs of 2-Methoxyestradiol, an Endogenous Mammalian Metabolite of Estradiol That Inhibits Tubulin Polymerization by Binding to the Colchicine Binding Site, J. Med Chem., 1995, 38, 2041.
   (c) Hamel, E.; Lin, C. M.; Flynn, E.; DÆAmato, R J. D., Interactions of 2-Methoxyestradiol, and Endogenous Mammalian Metabolite, with Unpolymerized Tubulin and with Tubulin Polymers, Biochemistry, 1996, 35, 1304.
   (d) Cushman, M.; He, H.-M.; Katzenellenbogen, J. A.; Varma, R. K.; Hamel, E.; Lin, C. M.; Ram, S.; Sachdeva, Y. P., Synthesis of Analogs of 2-Methoxyestradiol with Enhanced Inhibitory Effects on Tubulin Polymerization and Cancer Cell Growth, J. Med. Chem., 1997, (in press).
23) Boyd, M. R.; Paull, K. D., Some Practical Considerations and Applications of the National Cancer Institute In Vitro Anticancer Drug Discovery Screen, Drug Development Research, 1995, 34, 91.
24) Angerer, E.; Prekajac, J.; Strohmeier, J.; J. Med Chem. 1984, 27,1439-1447.
25) Inion, H.; Vogelaer, H.; Bauthier, J.; Colot, M.; Richard, J.; Eur. J. Med. Chem. : 1977, 5, 483-487.
26) Gastpar, R.; Goldbrunner, M.; Marko, D.; von Angerer, E., MethoxySubstituted 3-Formyl-2-phenylindoles Inhibit Tubulin Polymerization, J. Mod Chem. 1998, 41, 4965 -4972.
27) Pettit, G.R.; and Rhodes, M.R,Anti-Cancer Drug Des. 1998, 13, 183-191.
28) Dark, G.G.; Hill, S.A.; Prise, V.E.; Tozer, G.M.; Pettit, G.R.; and Chaplin, D.J., Cancer Res. 1997, 57; 1829-1834.
29) lyer, S.; Chaplin, D.J.; Rosenthal, D.S.; Boulares, A.H.; Li, L.Y.; and Smulson, M.E., Cancer Res. 1998, 58, 4510-4514.
30) Pinney et al., Tubulin Binding Ligands and Corresponding Prodrug Constructs, Provisional Patent Application, US Ser. No. 60/188,295, filed on March 10, 2000.
31) Pinney et al., Synthesis of a Benzo[b]thiophene-based Vascular Targeting Prodrug and Related Anti-Tubulin Ligands, 220th American Chemical Society, National Meeting, Division of Organic Chemistry, Abstract No. 196, Washington, D.C., August 20-24, 2000.

## Claims

1. A compound of the structure: 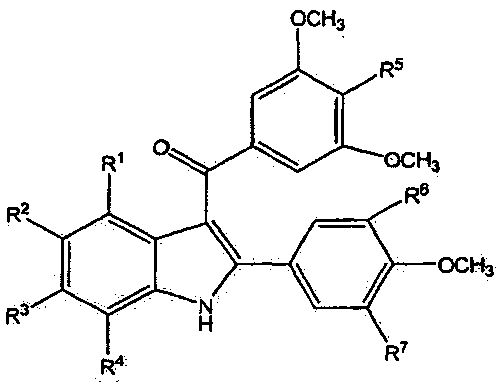 or a pharmaceutically-acceptable salt thereof, wherein:
R¹ or R³ is a methoxy group;
R⁵ is OR⁸, where R⁸ is hydrogen or an alkyl group; and:
I. the remaining R¹ to R⁷:
a. include at least one a phosphate ester moiety of the formula -OP(O)(O⁻ M⁺)₂, wherein M⁺ is a pharmaceutically-acceptable cation or a phosphoramidate of the formula -NP(O)(O⁻M⁺)₂, wherein M⁺ is a pharmaceutically-acceptable cation, or NP(O)(OR)₂, wherein R is an alkyl group with up to 8 carbon atoms, the groups being the same or different; or
b. when R⁵ is methoxy, include at least one amine group of the formula NH₂, NHR⁹ or NR⁹R¹⁰, in which R⁹ and R¹⁰ are the same or different and are selected from alkyl groups having up to 8 carbon atoms;
the rest of the remaining R¹ to R⁷ all being hydrogen; or
II. when R⁵ is methoxy, R⁶ or R⁷ is OH and the remaining R¹ to R⁷ are all hydrogen.

2. A compound according to Claim 1, wherein R¹ or R³ is a methoxy group, R⁵ is methoxy, R⁶ or R⁷ is OH and the rest of R¹ to R⁷ are all hydrogen.

3. A compound according to Claim 1, wherein R³ and R⁵ are methoxy.

4. A compound according to Claim 3, wherein at least one of R¹ to R⁷ is a phosphate ester moiety of the formula -OP(O)(O⁻M⁺)₂, wherein M⁺ is a pharmaceutically-acceptable cation.

5. A compound according to Claim 3, wherein R⁷ is said amine group and R¹, R², R⁴, and R⁶ are all hydrogen.

6. A compound according to Claim 5, wherein R⁷ is -NH₂.

7. A compound according to Claim 3, wherein R⁷ is said phosphate ester moiety, and R¹, R², R⁴ and R⁶ are all hydrogen.

8. A compound according to Claim 3, wherein k⁷ is said phosphoramidate and R¹, R², R⁴ and R⁶ are all hydrogen.

9. A compound according to Claim 8, wherein R⁷ is -NP(O)(OCH₂CH₃)₂.

10. A compound according to Claim 8, wherein R⁷ is -NP(O)(O⁻Nₐ⁺)₂.

11. A compound according to Claim 1, wherein R¹ and R⁵ are methoxy.

12. A compound according to Claim 11, wherein at least one of R¹ to R⁷ is a phosphate ester moiety of the formula -OP(O)(O⁻M⁺)₂, wherein M⁺ is a pharmaceutically-acceptable cation.

13. A compound according to Claim 11, wherein k⁷ is -OH, and R², R³, R⁴ and R⁶ are all hydrogen.

14. A compound according to Claim 11, wherein R⁷ is said amine group and R², R³, R⁴ and R⁶ are all hydrogen.

15. A compound according to Claim 14, wherein R⁷ is -NH₂.

16. A compound according to Claim 11, wherein R⁷ is said phosphate ester moiety, and R², R³, R⁴ and R⁶ are all hydrogen.

17. A compound according to Claim 16, wherein R⁷ is -OP(O)(O-Na⁺)₂.

18. A compound according to Claim 11, wherein k⁷ is said phosphoramidate and R², R³, R⁴ and R⁶ are all hydrogen.

19. A compound according to Claim 18, wherein R⁷ is -NP(O)(OCH₂CH₃)₂.

20. A compound according to Claim 18, wherein k⁷ is -NP(O)(O⁻Na⁺)₂.

21. A compound according to any preceding claim, for use in inhibiting tubulin polymerisation *in vitro* in a tubulin-containing system.

22. A compound according to any of Claims 1 to 20, for use in the treatment of a host afflicted with a neoplastic disease.

23. A compound according to any of Claims 1 to 20, for use in the treatment of a cancer of the group containing leukaemia, melanoma and lung, colon, thyroid, CNS, ovarian, renal, prostate and breast cancers.

24. A compound according to Claim 23, when dependent on Claim 7, wherein R⁷ is -OP(O)(O⁻Na⁺)₂.

25. A compound according to any of Claims 21 to 23, when dependent on Claim 2, wherein R³ and R⁵ are methoxy, k⁷ is OH and the rest of R¹ to k⁷ are all hydrogen.

26. A preparation for pharmaceutical use containing a compound according to any of Claims 1 to 25 as an active component along with a pharmaceutically-acceptable carrier.

## Patentansprüche

1. Verbindung mit der Struktur: 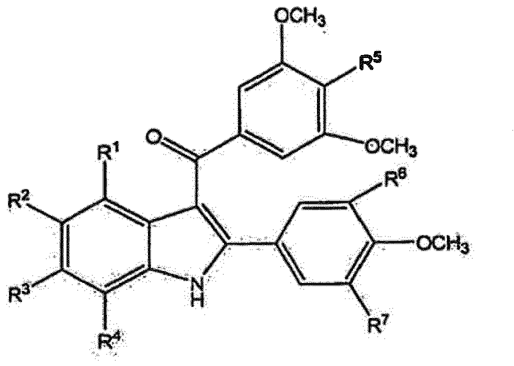 oder ein pharmazeutisch verträgliches Salz davon, worin:
R¹ oder R³ eine Methoxygruppe ist,
R⁵ OR⁸ ist, worin R⁸ Wasserstoff oder eine Alkylgruppe ist, und
I. die verbleibenden R¹ bis R⁷
a. mindestens eines von einem Phosphatesterrest mit der Formel -OP(O)(O-M^{k})₂, worin M⁺ ein pharmazeutisch verträgliches Kation ist, oder einem Phosphoramidat der Formel -NP(O)(O⁻M⁺)₂, worin M⁺ ein pharmazeutisch verträgliches Kation ist, oder -NP(O)(OR)₂, worin R eine Alkylgruppe mit bis zu 8 Kohlenstoffatomen ist, wobei die Gruppen gleich oder verschieden sind, umfassen, oder
b. wenn R⁵ Methoxy ist, mindestens eine Amingruppe mit der Formel NH₂, NHR⁹ oder NR⁹R¹⁰ umfassen, worin R⁹ und R¹⁰ gleich oder verschieden sind und aus Alkylgruppen mit bis zu 8 Kohlenstoffatomen ausgewählt sind,
wobei die übrigen der verbleibenden R¹ bis R⁷ alle Wasserstoff sind oder
II. wenn R⁵ Methoxy ist, R⁶ oder R⁷ OH ist und die verbleibenden R¹ bis R⁷ alle Wasserstoff sind.

2. Verbindung nach Anspruch 1, worin R¹ oder R³ eine Methoxygruppe ist, R⁵ Methoxy ist, R⁶ oder R⁷ OH ist und die übrigen R¹ bis R⁷ alle Wasserstoff sind.

3. Verbindung nach Anspruch 1, worin R³ und R⁵ Methoxy sind.

4. Verbindung nach Anspruch 3, worin mindestens einer von R¹ bis R⁷ ein Phosphatesterrest mit der Formel -OP(PO)(O⁻M⁺)₂ ist, worin M⁺ ein pharmazeutisch verträgliches Kation ist.

5. Verbindung nach Anspruch 3, worin R⁷ die Amingruppe ist und R¹, R², R⁴ und R⁶ alle Wasserstoff sind.

6. Verbindung nach Anspruch 5, worin R⁷ -NH₂ ist.

7. Verbindung nach Anspruch 3, worin R⁷ der Phosphatesterrest ist und R¹, R², R⁴ und R⁶ alle Wasserstoff sind.

8. Verbindung nach Anspruch 3, worin R⁷ das Phosphoramidat ist und R¹, R², R⁴ und R⁶ alle Wasserstoff sind.

9. Verbindung nach Anspruch 8, worin R⁷ -NP(O)(OCH₂CH₃)₂ ist.

10. Verbindung nach Anspruch 8, worin R⁷ -NP(O)(O⁻Na⁺)₂ ist.

11. Verbindung nach Anspruch 1, worin R¹ und R⁵ Methoxy sind.

12. Verbindung nach Anspruch 11, worin mindestens einer von R¹ bis R⁷ ein Phosphatesterrest mit der Formel -OP(O)(O⁻M⁺)₂ ist, worin M⁺ ein pharmazeutisch verträgliches Kation ist.

13. Verbindung nach Anspruch 11, worin R⁷ -OH ist und R², R³, R⁴ und R⁶ alle Wasserstoff sind.

14. Verbindung nach Anspruch 11, worin R⁷ die Amingruppe ist und R², R³, R⁴ und R⁶ alle Wasserstoff sind.

15. Verbindung nach Anspruch 14, worin R⁷ -NH₂ ist.

16. Verbindung nach Anspruch 11, worin R⁷ der Phosphatesterrest ist und R², R³, R⁴ und R⁶ alle Wasserstoff sind.

17. Verbindung nach Anspruch 16, worin R⁷ -OP(O)(O⁻Na⁺)₂ ist.

18. Verbindung nach Anspruch 11, worin R⁷ das Phosphoramidat ist und R², R³, R⁴ und R⁶ alle Wasserstoff sind.

19. Verbindung nach Anspruch 18, worin R⁷ -NP(O)(OCH₂CH₃)₂ ist.

20. Verbindung nach Anspruch 18, worin R⁷ -NP(O)(O⁻Na⁺)₂ ist.

21. Verbindung nach einem der vorhergehenden Ansprüche zur Verwendung bei der Hemmung der Tubulinpolymerisation *in vitro* in einem Tubulin-enthaltenden System.

22. Verbindung nach einem der Ansprüche 1 bis 20 zur Verwendung bei der Behandlung eines Wirts, der eine neoplastische Erkrankung aufweist.

23. Verbindung nach einem der Ansprüche 1 bis 20 zur Verwendung bei der Behandlung von Krebs aus der Gruppe, die Leukämie, Melanom und Lungen-, Kolon-, Schilddrüsen-, ZNS-, Eierstock-, Nieren-, Prostata- und Brustkrebs enthält.

24. Verbindung nach Anspruch 23, wenn dieser von Anspruch 7 abhängt, worin R⁷ -OP(O)(O⁻Na⁺)₂ ist.

25. Verbindung nach einem der Ansprüche 21 bis 23, wenn diese von Anspruch 2 abhängen, worin R³ und R⁵ Methoxy sind, R⁷ OH ist und die übrigen von R¹ bis R⁷ alle Wasserstoff sind.

26. Präparat zur pharmazeutischen Verwendung, das eine Verbindung nach einem der Ansprüche 1 bis 25 als Wirkstoff zusammen mit einem pharmazeutisch verträglichen Träger enthält.

## Revendications

1. Composé de structure : 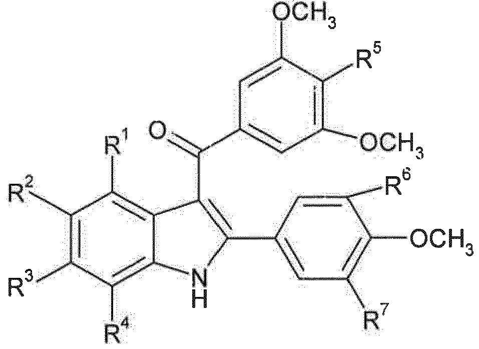 ou un sel pharmaceutiquement acceptable de celui-ci, structure dans laquelle :
R¹ ou R³ est un groupe méthoxy ;
R⁵ est OR⁸ où est l'hydrogène ou un groupe alkyle ; et
I. les R¹ à R⁷ restants :
a. comprennent au moins un fragment ester phosphate de formule -OP(O) (O⁻M⁺)₂ où M⁺ est un cation pharmaceutiquement acceptable ou un phosphoramidate de formule -NP(O)(O⁻M⁺)₂ où M⁺ est un cation pharmaceutiquement acceptable, ou -NP(O)(OR)₂ où R est un groupe alkyle ayant jusqu'à 8 atomes de carbone, les groupes étant identiques ou différents ; ou
b. quand R⁵ est méthoxy, comprennent au moins un groupe amine de formule NH₂, NHR⁹ ou NR⁹R¹⁰ où R⁹ et R¹⁰ sont identiques ou différents et sont choisis parmi les groupes alkyle ayant jusqu'à 8 atomes de carbone ;
tout le reste des R¹ à R⁷ restants étant l'hydrogène ; ou
II. quand R⁵ est méthoxy, R⁶ ou R⁷ est OH et les R¹ à R⁷ restants sont tous l'hydrogène.

2. Composé selon la revendication 1, dans lequel R¹ ou R³ est un groupe méthoxy, R⁵ est méthoxy, R⁶ ou R⁷ est OH et les R¹ à R⁷ restants sont tous l'hydrogène.

3. Composé selon la revendication 1, dans lequel R³ et R⁵ sont méthoxy.

4. Composé selon la revendication 3, dans lequel au moins l'un de R¹ à R⁷ est un fragment ester phosphate de formule -OP(O)(O⁻M⁺)₂ où M⁺ est un cation pharmaceutiquement acceptable.

5. Composé selon la revendication 3, dans lequel R⁷ est ledit groupe amine et R¹, R² R⁴ et R⁶ sont tous l'hydrogène.

6. Composé selon la revendication 5, dans lequel R⁷ est -NH₂.

7. Composé selon la revendication 3, dans lequel R⁷ est ledit fragment ester phosphate, et R¹, R², R⁴ et R⁶ sont tous l'hydrogène.

8. Composé selon la revendication 3, dans lequel R⁷ est ledit phosphoramidate, et R¹, R², R⁴ et R⁶ sont tous l'hydrogène.

9. Composé selon la revendication 8, dans lequel R⁷ est -NP(O)(OCH₂CH₃)₂.

10. Composé selon la revendication 8, dans lequel R⁷ est -NP(O)(O⁻Na⁺)₂.

11. Composé selon la revendication 1, dans lequel R¹ et R⁵ sont méthoxy.

12. Composé selon la revendication 11, dans lequel au moins l'un de R¹ à R⁷ est un fragment ester phosphate de formule -OP(O)(O⁻M⁺)₂ où M⁺ est un cation pharmaceutiquement acceptable.

13. Composé selon la revendication 11, dans lequel R⁷ est -OH, et R², R³, R⁴ et R⁶ sont tous l'hydrogène.

14. Composé selon la revendication 11, dans lequel R⁷ est ledit groupe amine, et R², R³, R⁴ et R⁶ sont tous l'hydrogène.

15. Composé selon la revendication 14, dans lequel R⁷ est -NH⁷.

16. Composé selon la revendication 11, dans lequel R⁷ est ledit fragment ester phosphate, et R², R³, R⁴ et R⁶ sont tous l'hydrogène.

17. Composé selon la revendication 16, dans lequel R⁷ est -OP (O) (O⁻Na⁺)₂.

18. Composé selon la revendication 11, dans lequel R⁷ est ledit phosphoramidate, et R², R³, R⁴ et R⁶ sont tous l'hydrogène.

19. Composé selon la revendication 18, dans lequel R⁷ est -NP (O) (OCH₂CH₃)₂.

20. Composé selon la revendication 18, dans lequel R⁷ est -NP (O) (O⁻Na⁺)₂.

21. Composé selon l'une quelconque des revendications précédentes, pour utilisation dans l'inhibition de la polymérisation de tubuline in vitro dans un système contenant de la tubuline.

22. Composé selon l'une quelconque des revendications 1 à 20, pour utilisation dans le traitement d'un hôte affligé d'une maladie néoplasique.

23. Composé selon l'une quelconque des revendications 1 à 20, pour utilisation dans le traitement d'un cancer du groupe comprenant les leucémies, les mélanomes et les cancers du poumon, du côlon, de la thyroïde, du SNC, de l'ovaire, du rein, de la prostate et du sein.

24. Composé selon la revendication 23 quand elle dépend de la revendication 7, dans lequel R⁷ est -OP(O)(O⁻Na⁺)2.

25. Composé selon l'une quelconque des revendications 21 à 23 quand elles dépendent de la revendication 2, dans lequel R³ et R⁵ sont méthoxy, R⁷ est OH et les R¹ à R⁷ restants sont tous l'hydrogène.

26. Préparation à usage pharmaceutique contenant un composé selon l'une quelconque des revendications 1 à 25 à titre de composant actif conjointement avec un véhicule pharmaceutiquement acceptable.
